# EUROPÄISCHE PATENTSCHRIFT

(11) **EP 2 147 665 B1**
(45) Veröffentlichungstag und Bekanntmachung des Hinweises auf die Patenterteilung: **10.04.2013**
(21) Anmeldenummer: 09009547.2
(22) Anmeldetag: 23.07.2009
(51) Int. Cl.: A61F 13/514

(54) **Hygieneprodukt**
Hygiene product
Produit hygiénique

(30) Priorität: 24.07.2008 DE 202008009906 U
(43) Veröffentlichungstag der Anmeldung: 27.01.2010
(73) Patentinhaber: W. Pelz GmbH & Co. KG, 23812 Wahlstedt/Holstein (DE)
(72) Erfinder: Bark, Andreas, 22397 Hamburg (DE); Schabram, Birte, 24576 Hagen (DE)
(74) Vertreter: Hauck Patent- und Rechtsanwälte

(56) Entgegenhaltungen:
- EP-A1- 1 468 661
- DE-U1-202006 009 469

## Beschreibung

Die Erfindung bezieht sich auf ein Hygieneprodukt für Ausscheidungen des menschlichen Körpers.

Hygieneprodukte für Ausscheidungen des menschlichen Körpers sind beispielsweise Binden, Dickbinden, Inkontinenzeinlagen, Inkontinenzvorlagen, Inkontinenzpants, Slipeinlagen, (Wöchnerinnen-)Vorlagen, Höschenwindeln und Stilleinlagen. Bekannte Hygieneprodukte weisen eine Wäscheschutzschicht und eine darauf angeordnete Absorptionsschicht auf, die an der von der Wäscheschutzschicht abgewandten Seite mit einer flüssigkeitsdurchlässigen Abdeckung versehen sein kann. Das Hygieneprodukt wird mit der von der Wäscheschutzschicht abgewandten Seite an den Körper des Menschen angelegt, dort, wo Körperflüssigkeit austritt. Die Körperflüssigkeit gelangt - gegebenenfalls durch die Abdeckung hindurch - in die Absorptionsschicht hinein und wird von der Wäscheschutzschicht von der Kleidung der Benutzerin abgehalten.

Bei herkömmlichen Hygieneprodukten wird die Wäscheschutzschicht von einer Folie aus Kunststoff gebildet. Diese Folie an der körperabgewandten Außenseite des Hygieneproduktes wird bei der Benutzung vielfach als störend empfunden.

Ferner sind Hygieneprodukte bekannt, bei denen die Wäscheschutzschicht durch ein von einer Folie kaschiertes Nonwoven gebildet ist. Die Folie ist verhältnismäßig dick, damit sie beim kontinuierlichen Herstellungsprozess des Laminats, bei dem sie von einer Rolle abgewickelt wird, nicht reißt. In der Regel sind die verschiedenen Lagen des Laminats so miteinander verbunden, dass sie sich nicht lösen und gegeneinander verschieben können. Trotzdem kann eine verhältnismäßig dicke Folie, die auflaminiert ist, als störend empfunden werden. Falls sich die Lagen gegeneinander verschieben, kann das Hygieneprodukt beim Tragen rascheln, was ebenfalls als störend empfunden wird.

Ferner bekannt ist der Einsatz von hydrophoben Spinnvliesen als Wäscheschutzschicht. Diese Spinnvliese sind luftdurchlässig und sperren Flüssigkeit. Sie können jedoch unter erhöhtem Wasserdruck oder bei Einsatz von Duftölen in Verbindung mit Emulgatoren durchlässig werden.

Aus der DE 20 2006 009 469 U1 ist ein Hygieneprodukt zum Aufnehmen von Ausscheidungen des menschlichen Körpers mit einer Wäscheschutzschicht umfassend ein Laminat aus einem Nonwoven und einem auf der Innenseite des Nonwovens angeordneten geschlossenen flüssigkeitsundurchlässigen Film aus einem Schmelzklebstoff und einer auf dem Film angeordnete Absorptionsschicht. Das Laminat ist zuverlässig dicht, hat eine stoffartige Oberfläche, kann dünn ausgeführt sein, ist geräuschmindernd und preisgünstig. Bei der Herstellung des Laminats mit nur einem Film aus einem Schmelzklebstoff können Fehlstellen und damit ein totales Versagen des Hygieneproduktes nicht sicher ausgeschlossen werden. Gemäß einer Ausgestaltung umfasst der Film mehrere aufeinander aufgebrachte bzw. aufeinander extrudierte Schichten aus Schmelzklebstoff. Der Auftrag in mehreren Schichten ermöglicht sehr geringe Auftragsstärken bzw. Flächengewichte in jeder einzelnen Schicht, wobei in den Schichten vorhandene Fehlstellen von mindestens einer weiteren Schicht abgedeckt werden. In der Summe sind hierdurch sehr geringe Auftragsgewichte bzw. Flächengewichte möglich. Wird auf einen ersten Film aus Schmelzklebstoff ein zweiter Film aus Schmelzklebstoff aufgetragen, so kann der zweite Film den ersten Film beschädigen, sodass keine Dichtigkeit erzielt wird. Die Herstellung eines sicheren Hygieneproduktes der vorbeschriebenen Art ist somit problematisch.

Davon ausgehend liegt der Erfindung die Aufgabe zugrunde, ein Hygieneprodukt zur Verfügung zu stellen, das zuverlässiger zur Wäscheseite hin abgedichtet ist und günstige Benutzungseigenschaften aufweist.

Die Aufgabe wird durch ein Hygieneprodukt mit den Merkmalen des Anspruchs 1 gelöst.

Vorteilhafte Ausgestaltungen des Hygieneproduktes sind in den Unteransprüchen angegeben.

Das erfindungsgemäß Hygieneprodukt zum Aufnehmen von Ausscheidungen des menschlichen Körpers hat eine Wäscheschutzschicht umfassend ein Laminat aus einem Nonwoven, einem auf der Innenseite des Nonwovens angeordneten Film aus einem Schmelzklebstoff, einem auf der Innenseite des Films angeordneten weiteren Nonwoven und einem auf der Innenseite des weiteren Nonwovens angeordneten weiteren Film aus einem Schmelzklebstoff und eine auf der Innenseite des weiteren Films angeordnete Absorptionsschicht.

Bei dem erfindungsgemäßen Hygieneprodukt ist die Wäscheschutzschicht ein Laminat, bei dem von außen nach innen abwechselnd Lagen eines Nonwovens und eines Filmes aus Schmelzklebstoff gebildet ist. Dabei umfasst das Laminat mindestens zwei Lagen des Nonwovens und mindestens zwei Filme aus Schmelzklebstoff. Da auf ein auf einem Nonwoven aufgebrachten Film aus einem Schmelzklebstoff ein weiteres Nonwoven aufgebracht und erst darauf ein weiterer Film aus Schmelzklebstoff aufgebracht wird, wird vermieden, dass bei der Herstellung der Auftrag des weiteren Filmes aus Schmelzklebstoff den erstgenannten Film aus Schmelzklebstoff zerstört. Durch die mehreren übereinander angeordneten Filme aus Schmelzklebstoff werden Fehlstellen in den einzelnen Filmen aus Schmelzklebstoff abgedeckt, sodass die Wäscheschutzschicht insgesamt mit großer Sicherheit flüssigkeitsundurchlässig ist.

Das erfindungsgemäße Laminat kann mit dünnen Lagen aus Nonwoven und dünnen Filmen aus Schmelzklebstoff hergestellt werden, sodass es ein geringes Flächengewicht aufweist und sehr flexibel ist. Die Wahrscheinlichkeit, dass sich Fehlstellen in den verschiedenen Filmen überlagern und zu einer Undichtigkeit der Wäscheschutzschicht führen, ist äußerst gering. In der Regel befinden sich nämlich die Fehlstellen versetzt in der Fläche. Infolgedessen kann sogar gezielt in Kauf genommen werden, dass die Filme aus Schmelzklebstoff Fehlstellen haben, sodass sehr dünne Filme mit geringen Flächengewichten möglich sind. Infolgedessen kann die Wäscheschutzschicht mit großer Flexibilität und insgesamt mit geringem Flächengewicht ausgeführt werden.

Die Filme aus Schmelzklebstoff vermitteln kein unangenehmes Tragegefühl, da sie an der Innenseite des äußeren Nonwovens angeordnet sind. Die körperabgewandte Außenseite des Hygieneproduktes hat aufgrund des dort angeordneten Nonwovens eine textile Oberfläche und wird als angenehm empfunden. Bei Ausführung des Laminats mit geringer Stärke und damit geringem Flächengewicht sind Hygieneprodukte mit geringer Stärke möglich, die beim Tragen weniger stören. Das Laminat und das Hygieneprodukt ist sehr kostengünstig herstellbar. Dabei ist besonders vorteilhaft und kostenmindernd für die Produktion, dass der Schmelzklebstoff "inline" bzw. bei der Produktion des Wäscheschutzes auf das Nonwoven aufgebracht werden kann. Ferner ist vorteilhaft, dass eine anschließend aufgebrachte Absorptionsschicht durch den abnehmenden Schmelzklebstoff mit dem Laminat verbindbar ist. Dann ist der innere Film aus Schmelzklebstoff zugleich Teil des Wäscheschutzes und Konstruktionskleber.

Gemäß einer Ausgestaltung weist die Wäscheschutzschicht mindestens eine weitere Anordnung aus einem Nonwoven mit einem Film auf der Innenseite auf, wobei das Nonwoven der weiteren Anordnung mit der Außenseite an der Innenseite eines Films angeordnet ist und der Film der weiteren Anordnung mit der Innenseite an einer Absorptionsschicht oder an der Außenseite des Nonwovens eine weiteren Anordnung aus Nonwoven und Film angeordnet ist. Die weitere Anordnung aus Nonwoven und Film aus Schmelzklebstoff verbessert weiterhin die Flüssigkeitsdichtigkeit der Wäscheschutzschicht und damit die Sicherheit des Hygieneproduktes. Außerdem trägt sie dazu bei, dass die Dicke des Films aus Schmelzklebstoff weiter reduziert werden kann.

Gemäß einer Ausgestaltung hat mindestens ein Film aus Schmelzklebstoff ein Flächengewicht im Bereich von 1 bis 20 g/m2. Gemäß einer weiteren Ausgestaltung hat mindestens ein Film aus Schmelzklebstoff ein Flächengewicht im Bereich von 5 bis 10 g/m2. Bevorzugt haben sämtliche Filme aus Schmelzklebstoff ein Flächengewicht in den vorgenannten Bereichen.

Gemäß einer weiteren Ausgestaltung hat mindestens ein zwischen äußerem Nonwoven und Absorptionsschicht angeordnetes weiteres Nonwoven ein geringeres Flächengewicht als das äußere Nonwoven. Die stärkere Ausführung des äußeren Nonwovens kommt einem angenehmen Tragegefühl zugute. Für die Vermeidung einer Beschädigung eines Films aus Schmelzklebstoff reicht es aus, wenn das darauf angeordnete weitere Nonwoven ein verhältnismäßig geringes Flächengewicht aufweist.

Bevorzugt hat das äußere Nonwoven ein Flächengewicht von 10 bis 30 g/m2. Weiterhin bevorzugt hat es ein Flächengewicht in dem Bereich von 15 bis 25 g/m2, insbesondere von etwa 21 g/m2.

Gemäß einer weiteren Ausgestaltung hat mindestens ein zwischen äußerem Nonwoven und Absorptionsschicht angeordnetes weiteres Nonwoven ein Flächengewicht von 5 bis 15 g/m2.. Weiterhin bevorzugt hat es ein Flächengewicht von etwa 7 bis 9 g/m2, insbesondere von etwa 8 g/m2.

Ein Nonwoven ist grundsätzlich ein textiles Flächengebilde aus verfestigten Fasern. Das Laminat kann insbesondere ein wasserstrahlverfestigtes oder ein nadelverfestigtes oder ein bindemittelverfestigtes Nonwoven aufweisen. Gemäß einer Ausgestaltung weist das Laminat ein thermoverfestigtes Nonwoven auf. Ein thermoverfestigtes Nonwoven umfasst Kunststofffasern, die bei der Herstellung des Nonwovens zumindest an der Oberfläche aufgeschmolzen werden, sodass nach dem Abkühlen an den Kontaktstellen feste Verbindungen entstehen. Die Verfestigung wird durch ein punktförmiges Verschweißen der Fasern im Wesentlichen über den gesamten Querschnitt des Nonwovens bewirkt. Die Struktur des thermisch verfestigten Nonwovens ist durch die Verschweißung der Fasern geprägt. Thermisch verfestigte Nonwoven werden z.B. hergestellt, indem als Ballenwaren bereitgestellte Polypropylenfasern aufgelöst, als Flor abgelegt und durch temperierte Kalander hindurchgeführt werden, wobei die Fasern an- bzw. verschmelzen und sich nach dem Abkühlen ein thermisch verfestigtes Nonwoven aus Polypropylen bildet. Typischerweise werden thermisch verfestigte Nonwoven aus PP-Fasern hergestellt. Es werden aber auch Bikomponenten-Fasern (innen PP und außen PE) und verstärkt PE-Fasern eingesetzt.

Gemäß einer anderen Ausgestaltung ist das Nonwoven ein Spinnvlies. Ein Spinnvlies wird z.B. hergestellt, indem mittels einer Spinndüse aus aufgeschmolzenem Kunststoff dünne Fasern hergestellt werden, die von einem starken Luftstrom auf einem Band abgelegt werden, wo sie durch thermisches Verbinden miteinander verbunden werden.

Gemäß einer bevorzugten Ausgestaltung ist das äußere Nonwoven ein thermisch verfestigtes Nonwoven auf der Basis synthetischer Stapelfasern. Gemäß einer weiteren Ausgestaltung ist mindestens ein zwischen dem äußeren Nonwoven und der Absorptionsschicht angeordnetes weiteres Nonwoven ein Spunbond.

Gemäß einer Ausgestaltung ist der Schmelzklebstoff ein druckempfindlicher Schmelzklebstoff. Derartige Schmelzklebstoffe werden beispielsweise an Klebestellen zur Befestigung an der Kleidung an den körperabgewandten Seiten von (Damen-) Binden und Slipeinlagen eingesetzt. Diese Klebestellen sind vor Benutzung durch eine Schutzschicht aus Silikonpapier abgedeckt, das vor Anbringung des Hygieneproduktes abgezogen wird.

Gemäß einer Ausgestaltung ist der innerste Film aus Schmelzklebstoff durch den Schmelzklebstoff mit der Absorptionsschicht verklebt. Der Schmelzklebstoff dient somit sogleich der Abdichtung und dem Zusammenhalt verschiedener Schichten des Hygieneproduktes.

Die Absorptionsschicht kann verschieden beschaffen sein. Gemäß einer Ausgestaltung umfasst sie ein Fluffkissen oder ein Airlaid mit oder ohne enthaltenen Superabsorber. Bei dem Fluffkissen handelt es sich z.B. um zu einem Kissen geformte Zellulosefasern. Ein Airlaid besteht z.B. aus vom Luftstrom auf einem Sieb abgelegte und bei Vakuum von unten unter Verfestigung durch Bindemittel oder Aufheizen bei enthaltenen Schmelzfasern hergestelltem Zellulosematerial. Das Airlaid kann auch superabsorbierende Substanzen wie Superabsorber (SAP) enthalten.

Gemäß einer Ausgestaltung umfasst die Absorptionsschicht Zellulosefasern und/oder Baumwollfasern und/oder Viskosefasern.

Grundsätzlich ist es möglich, dass die Absorptionsschicht direkt mit der Oberseite an den Körper der Benutzerin angelegt wird. Ferner ist es möglich, dass die Fasern an der Außenseite der Absorptionsschicht bzw. die Absorptionsschicht in Oberflächennähe verdichtet (z.B. durch Prägen) oder wasservernadelt oder anderweitig verfestigt werden, um einen Austritt von Fasern an der Außenseite entgegenzuwirken bzw. um ein angenehmes Tragegefühl zu vermitteln. Gemäß einer Ausgestaltung ist auf der Außenseite der Absorptionsschicht eine flüssigkeitsdurchlässige Abdeckung angeordnet. Die Abdeckung ist z.B. aus einem anderen Material als die Absorptionsschicht mit einem geeigneten Griff, das sich auf der Haut gut anfühlt. Ferner ist es möglich, durch eine geeignete Ausführung der Abdeckung den Flüssigkeitsstrom in die Absorptionsschicht hineinzuleiten und zu verhindern, dass Flüssigkeit aus der Absorptionsschicht durch die Abdeckung hindurch nach außen tritt.

Die flüssigkeitsdurchlässige Abdeckung z.B. durch einen stellenweise oder punktuell aufgebrachten Kleber mit der Außenseite und/oder dem Rand der Absorptionsschicht verbunden. Zusätzlich oder stattdessen kann die flüssigkeitsdurchlässige Abdeckung um die Ränder der Absorptionsschicht geschlagen und zumindest randseitig unterhalb der Absorptionsschicht mit dem Film aus Schmelzklebstoff an der Oberseite der Wäscheschutzschicht verbunden sein. Hierbei kann der Film aus Schmelzklebstoff zugleich einen Konstruktionskleber bilden, der die flüssigkeitsdurchlässige Abdeckung fixiert.

Gemäß einer Ausgestaltung ist die Abdeckung eine Lochfolie und/oder ein perforiertes oder nicht perforiertes Nonwoven. Eine Lochfolie ist eine mit einer Vielzahl Löchern versehene Folie aus einem Kunststoff. Durch die Gestaltung der Löcher bzw. Perforation kann ggfs. die Richtung des Flüssigkeitsstroms gesteuert werden.

Gemäß einer Ausgestaltung sind verschiedene Schichten des Hygieneproduktes durch Klebstoff (z.B. Schmelzklebstoff) miteinander verbunden. Gemäß einer weiteren Ausgestaltung sind verschiedene Schichten des Hygieneproduktes miteinander versiegelt und/oder verprägt. Diese Ausgestaltungen betreffen die Verbindung des Laminats mit der Absorptionsschicht und/oder des Laminats und/oder der Absorptionsschicht mit der Abdeckung. Es sind auch Kombinationen der verschiedenen Verbindungsweisen möglich. Auch ist es möglich, die Absorptionsschicht ohne besondere zusätzliche Verbindung zwischen der Wäscheschutzschicht und der randseitig mit dieser verbundenen Abdeckung zu fixieren.

Gemäß einer Ausgestaltung ist das Hygieneprodukt eine Binde, Dickbinde, Inkontinenzeinlage, Inkontinenzvorlage, Inkontinenzpant, Slipeinlage, (Wöchnerinnen-) Vorlage, (Höschen-) Windel oder Stilleinlage.

Gemäß einer Ausgestaltung ist mindestens ein Nonwoven ein hydrophob oder hydrophob eingestelltes Nonwoven. Es wurde festgestellt, dass es für die Herstellung bzw. Dichtigkeit der Wäscheschutzschicht vorteilhaft ist, ein hydrophobes bzw. hydrophob eingestelltes Nonwoven bzw. Vlies einzusetzen. Das Nonwoven ist insbesondere hydrophob, wenn es aus hydrophoben Fasern hergestellt ist. Es ist insbesondere hydrophob eingestellt, wenn es durch Behandlung mit geeigneten Stoffen bzw. Überzügen bzw. Lösungsmitteln hydrophobisiert wurde. Die Erfindung wird nachfolgend anhand der anliegenden Zeichnung erläutert, die eine Vorrichtung zum Herstellen von Hygieneprodukten in einem Vertikalschnitt zeigt.

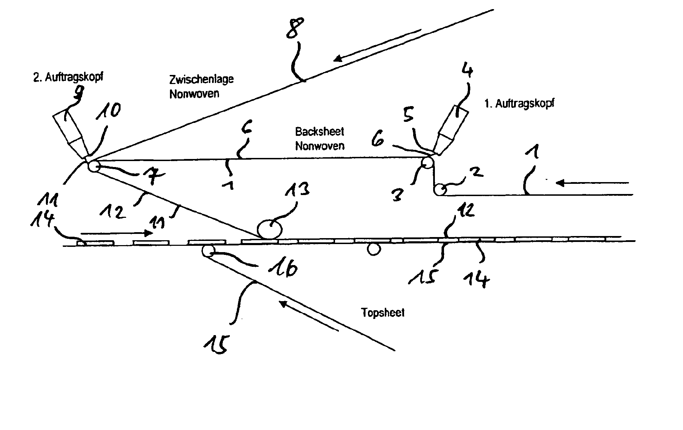

Die in der Zeichnung dargestellte Vorrichtung dient der Produktion von Slipeinlagen oder anderen Hygieneprodukten.

Der Vorrichtung wird ein Nonwoven 1 in Form eines endlosen Bahnmaterials zugeführt. Dieses wird über erste und zweite Umlenkrollen 2,3 an einen Auftragskopf 4 mit einer Schlitzdüse 5 vorbeigeführt, durch den ein Film 6 eines Schmelzklebers im schmelzflüssigen Zustand auf das Nonwoven 1 im Bereich der Umlenkrolle 3 aufgebracht wird, von der aus das Bahnmaterial horizontal abgezogen wird.

Das mit dem Film 6 beschichtete Nonwoven 1 gelangt dann auf eine weitere Umlenkrolle 7, auf die außen eine weitere Lage Nonwoven 8 in Form eines endlosen Bahnmaterials aufgebracht wird. Im Bereich dieser weiteren Umlenkrolle wird mittels eines weiteren Auftragskopfes 9, der eine Schlitzdüse 10 aufweist, ein weiterer Film 11 aus Schmelzklebstoff im schmelzflüssigen Zustand aufgebracht. Im Bereich der weiteren Umlenkrolle 10 verbinden sich die weitere Lage Nonwoven 8 und das erstgenannte Nonwoven 1 durch den auf diesen aufgebrachten Film 6 aus Schmelzklebstoff miteinander.

Das Laminat 12 aus Nonwoven 1, Film 6, weiterem Nonwoven 8 und Film 11 wird über eine weitere Umlenkrolle 13 auf Absorptionskissen 14 aufgebracht, die in einer endlosen Kette von einer horizontalen Transporteinrichtung an der Unterseite der weiteren Umlenkrolle 13 vorbei transportiert werden. Von unten wird auf die endlose Kette aus Absorptionskissen 14 eine flüssigkeitsdurchlässige Abdeckschicht 15 in Form eines endlosen Bahnmaterials z.B. aus Lochfolie oder Nonwoven über eine weitere Umlenkrolle 16 aufgebracht.

Die von oben mit dem Laminat 12 und von unten mit der Abdeckschicht 15 beschichteten Absorptionskissen 14 werden einer nicht gezeigten Schneidvorrichtung zugeführt, die die Hygieneprodukte vereinzelt. Im Bereich dieser Schneidvorrichtung kann die Abdeckschicht 15 mit seitlich überstehenden Rändern des Laminats 12 verklebt werden. Hierbei wird die Klebewirkung der auf das weitere Nonwoven 8 aufgebrachten Schicht 11 aus Schmelzklebstoff sowohl für die Verbindung des Laminats 12 mit den Absorptionskissen 14 als auch für die Verbindung der Abdeckschicht 15 mit dem Laminat 12 genutzt.

Der vereinzelten Hygieneprodukte können in eine Schutzfolie eingepackt und an den Verbraucher geliefert werden.

## Patentansprüche

1. Hygieneprodukt zum Aufnehmen von Ausscheidungen des menschlichen Körpers mit einer flüssigkeitsundurchlässigen Wäscheschutzschicht umfassend ein Laminat (12) aus einem Nonwoven (1), einem auf der Innenseite des Nonwovens (1) angeordneten Film (6) aus einem Schmelzklebstoff, einem auf der Innenseite des Films (6) angeordneten weiteren Nonwoven (8) und einem auf der Innenseite des weiteren Nonwovens (8) angeordneten weiteren Film (11) aus einem Schmelzklebstoff und einer auf der Innenseite des weiteren Films (11) angeordneten Absorptionsschicht (14).

2. Hygieneprodukt nach Anspruch 1, bei dem die Wäscheschutzschicht mindestens eine weitere Anordnung aus einem Nonwoven mit einem Film aus Schmelzklebstoff auf der Innenseite aufweist, wobei das Nonwoven der weiteren Anordnung mit der Außenseite an der Innenseite eines Films (11) aus Schmelzklebstoff angeordnet ist und der Film aus Schmelzklebstoff der weiteren Anordnung mit der Innenseite an einer Absorptionsschicht (14) oder an der Außenseite des Nonwovens einer weiteren Anordnung aus Nonwoven und Film aus Schmelzklebstoff angeordnet ist.

3. Hygieneprodukt nach Anspruch 1 oder 2, bei dem mindestens ein Film (6, 11) aus Schmelzklebstoff ein Flächengewicht im Bereich von 1 bis 20 g/m2 aufweist.

4. Hygieneprodukt nach Anspruch 3, bei dem mindestens ein Film (6, 11) aus Schmelzklebstoff ein Flächengewicht im Bereich von 5 bis 10 g/m2 aufweist.

5. Hygieneprodukt nach einem der Ansprüche 1 bis 4, bei dem mindestens ein zwischen äußerem Nonwoven (1) und Absorptionsschicht (14) angeordnetes weiteres Nonwoven (8) ein geringeres Flächengewicht als das äußere Nonwoven (1) aufweist.

6. Hygieneprodukt nach einem der Ansprüche 1 bis 5, bei dem das äußere Nonwoven (1) ein Flächengewicht von 10 bis 30 g/m2 aufweist.

7. Hygieneprodukt nach Anspruch 6, bei dem mindestens ein zwischen äußerem Nonwoven (1) und Absorptionsschicht (14) angeordnetes weiteres Nonwoven (8) ein Flächengewicht von 5 bis 15 g/m2 aufweist.

8. Hygieneprodukt nach einem der Ansprüche 1 bis 7, bei dem das äußere (1) Nonwoven ein thermisch verfestigtes Nonwoven auf der Basis synthetischer Stapelfasern ist.

9. Hygieneprodukt nach einem der Ansprüche 1 bis 8, bei dem mindestens ein zwischen dem äußeren Nonwoven (1) und der Absorptionsschicht (14) angeordnetes weiteres Nonwoven (8) ein Spunbond ist.

10. Hygieneprodukt nach einem der Ansprüche 1 bis 9, das eine Binde, Dickbinde, Inkontinenzeinlage, Inkontinenzvorlage, Inkontinenzpants, Slipeinlage (Wöchnerinnen), Vorlage, (Höschen-)Windel oder Stilleinlage ist.

11. Hygieneprodukt nach einem der Ansprüche 1 bis 10, bei dem mindestens ein Nonwoven ein hydrophob oder hydrophob eingestelltes Nonwoven ist.

## Claims

1. A hygiene product for absorbing excretions of the human body, with a liquid-impermeable underwear protection layer, comprising a laminate (12) from a non-woven (1), a film (6) of a hot-melt type adhesive, disposed on the inner side of the non-woven (1), a further non-woven (8) disposed on the inner side of the film (6) and a further film (11) of a hot-melt type adhesive disposed on the inner side of the further non-woven (8), and an absorption layer (14) disposed on the inner side of the further film (11).

2. The hygiene product according to claim 1, wherein the underwear protection layer has at least one further assembly from a non-woven with a film of hot-melt type adhesive on the inner side, wherein the non-woven of the further assembly is disposed on the inner side of a film (11) of hot-melt type adhesive with its outer side, and the film of hot-melt type adhesive of the further assembly is disposed on an absorption layer (14) with its inner side, or on the outer side of the non-woven of a further assembly from non-woven and film of hot-melt type adhesive.

3. A hygiene product according to claim 1 or 2, wherein at least one film (6, 11) of hot-melt type adhesive has a mass per unit area in the range of 1 to 20 g/m²

4. The hygiene product according to claim 3, wherein at least one film (6, 11) of hot-melt type adhesive has a mass per unit area in the range of 5 to 10 g/m².

5. A hygiene product according to any one of claims 1 to 4, wherein at least one further non-woven (8), situated between the outer non-woven (1) and the absorption layer (14), has a smaller mass per unit area than the outer non-woven (1).

6. A hygiene product according to any one of claims 1 to 5, wherein the outer non-woven (1) has a mass per unit area of 10 to 30 g/m².

7. The hygiene product according to claim 6, wherein at least one further non-woven (8), disposed between the outer non-woven (1) and the absorption layer (14), has a mass per unit area of 5 to 15 g/m².

8. A hygiene product according to any one of claims 1 to 7, wherein the outer non-woven (1) is a heat bonded non-woven on the basis of synthetic staple fibres.

9. A hygiene product according to any one of claims 1 to 8, wherein at least one further non-woven (8), disposed between the outer non-woven (1) and the absorption layer (14), is a spunbond.

10. A hygiene product according to any one of claims 1 to 9, which is a tie, thick tie, incontinence insertion pad, incontinence bed protection pad, incontinence pant, slip insert for women in childbed, pad, (panty)-diaper or breastfeeding insert.

11. A hygiene product according to any one of claims 1 to 10, wherein at least one non-woven is a hydrophobic or adjusted to be hydrophobic non-woven.

## Revendications

1. Produit hygiénique pour absorber des excrétions du corps humain, avec une couche de protection de dessous imperméable pour liquide, comprenant un aggloméré laminé (12) d'un non tissé (1), un film (6) d'une colle fusible disposé sur le côté intérieur du non tissé (1), un autre non tissé (8) disposé sur le côté intérieur du film (6) et un autre film (11) d'une colle fusible disposé sur le côté intérieur de l'autre non tissé (8), et une couche d'absorption (14) disposée sur le côté intérieur de l'autre film (11).

2. Produit hygiénique selon la revendication 1, dans lequel la couche de protection de dessous a au moins un autre assemblage d'un non tissé avec un film de colle fusible sur le côté intérieur, le non tissé de l'autre assemblage étant disposé sur le côté intérieur d'un film (11) de colle fusible par son côté extérieur, et le film de colle fusible de l'autre assemblage étant disposé sur une couche d'absorption (14) par son côté intérieur, ou sur le côté extérieur du non tissé d'un autre assemblage de non tissé et film de colle fusible.

3. Produit hygiénique selon la revendication 1 ou 2, dans lequel au moins un film (6, 11) de colle fusible a un grammage dans le domaine de 1 à 20 g/m².

4. Produit hygiénique selon la revendication 3, dans lequel au moins un film (6, 11) d'une colle fusible a un grammage dans le domaine de 5 à 10 g/m².

5. Produit hygiénique selon l'une quelconque des revendications 1 à 4, dans lequel au moins un autre non tissé (8), disposé entre le non tissé (1) extérieur et la couche d'absorption (14), an un grammage plus petit que celui du non tissé extérieur (1).

6. Produit hygiénique selon l'une quelconque des revendications 1 à 5, dans lequel le non tissé extérieur (1) a un grammage de 10 à 30 g/m².

7. Produit hygiénique selon la revendication 6, dans lequel au moins un autre non tissé (8), disposé entre le non tissé extérieur (1) et la couche d'absorption (14), a un grammage de 5 à 15 g/m².

8. Produit hygiénique selon l'une quelconque des revendications 1 à 7, dans lequel le non tissé extérieur (1) est un non tissé enduit par chaleur sur la base de fibres discontinues synthétiques.

9. Produit hygiénique selon l'une quelconque des revendications 1 à 8, dans lequel au moins un autre non tissé (8), disposé entre le non tissé extérieur (1) et la couche d'absorption (14), est un non tissé filé-lié.

10. Produit hygiénique selon l'une quelconque des revendications 1 à 9, qui est un bandeau, un bandeau épais, insert d'incontinence, drap protecteur d'incontinence, collant d'incontinence, insert de cache-sexe, drap protecteur pour accouchées, (collant)-lange ou insert de tétée.

11. Produit hygiénique selon l'une quelconque des revendications 1 à 10, dans lequel au moins un non tissé est un non tissé hydrophobique ou ajusté pour être hydrophobique.
